# EUROPÄISCHE PATENTANMELDUNG

(11) **EP 4 197 464 A1**
(43) Veröffentlichungstag der Anmeldung: **21.06.2023**
(21) Anmeldenummer: 21215570.9
(22) Anmeldetag: 17.12.2021
(51) Int. Cl.: A61B 17/70, A61B 17/80, A61B 5/00, A61B 17/00, G01N 27/72

(54) **MEDIZINISCHES KNOCHENIMPLANTAT SOWIE VERFAHREN ZUR ÜBERWACHUNG DES ZUSTANDS EINES IMPLANTATS**

(71) Anmelder: icotec AG, 9450 Altstätten (CH)
(72) Erfinder: VODERMAYER, Albert Maria, 8305 Dietlikon (CH); HÜPPI, Ramon, 9437 Marbach (CH); HEIM, Sebastian, 9000 St. Gallen (CH); STADLER, Roger Heinrich, 9052 Niederteufen (CH); DÜNKI, Andreas Christian, 8400 Winterthur (CH)
(74) Vertreter: Rutz, Andrea

(57) **Zusammenfassung**

Es wird ein medizinisches Knochenimplantat (1; 11, 14) zur Stabilisierung eines Knochenbereiches angegeben, welches einen aus einem thermoplastischen Kompositmaterial hergestellten Implantatkörper zur Verankerung in oder an einem menschlichen oder tierischen Knochen zwecks Stabilisierung des Knochenbereiches aufweist sowie zumindest einen Sensor (2) zur Messung von einem oder mehreren Parametern des Heilungsverlaufes und/oder eines Zustands des Implantats (1; 11, 14). Beim zumindest einen Sensor (2) handelt es sich um einen passiven magnetoelastischen Sensor (2). Ausserdem wird ein Verfahren zur Überwachung des Zustands eines Implantats (1; 11, 14) angegeben. Dabei wird ein passiver magnetoelastischer Sensor (2) zur Überwachung des Zustands des Implantats (1; 11, 14) während dessen Herstellung und/oder während dessen Lagerung verwendet.

## Beschreibung

### TECHNISCHES GEBIET

Die vorliegende Erfindung betrifft ein medizinisches Knochenimplantat zur Stabilisierung eines Knochenbereiches mit einem aus einem thermoplastischen Kompositmaterial hergestellten Implantatkörper, wie es insbesondere zur Versteifung der Wirbelsäule oder in der Traumabehandlung oft verwendet wird. Ausserdem betrifft die Erfindung ein Verfahren zur Überwachung des Zustands eines Implantats.

### STAND DER TECHNIK

Aus verschiedensten Gründen und immer öfters werden in der Medizin Implantate verwendet. Bei einem Implantat handelt es sich um eine künstliche Vorrichtung, die permanent oder für einen bestimmten, meist längeren Zeitraum in den menschlichen oder tierischen Körper eingesetzt wird. Das Implantat kann beispielsweise dazu dienen, eine Körperfunktion zu unterstützen oder zu ersetzen, wie dies zum Beispiel bei Herzschrittmachern, Cochlea-Implantaten oder Prothesen der Fall ist. Implantate können aber auch dazu dienen, ein zerstörtes Körperteil zu ersetzen oder zu vergrössern (plastische Chirurgie), oder den Träger zu überwachen, was beispielsweise mit RFID-Chips bei Haustieren oft gemacht wird.

Eine wichtige Gruppe von Implantaten sind Knochenimplantate, welche insbesondere zur Versteifung der Wirbelsäule und in der Traumabehandlung oft zum Einsatz kommen. Knochenimplantate sind zumindest teilweise, meist vollständig, innerhalb des Körpers angeordnet und sind dabei in oder an einem Knochen verankert, das heisst daran befestigt.

Bei Implantaten generell und bei Knochenimplantaten im Besonderen ist der Zustand des Implantats sowie der Heilungsverlauf des Patienten während des gesamten Einsatzes des Implantates im Körper von Interesse. Der Zustand des Implantats kann beispielsweise Verformungen des Implantats und somit auf das Implantat einwirkende Kräfte betreffen, anhand welcher Rückschlüsse auf den Heilungsverlauf gezogen werden können. Umgekehrt kann der Heilungsverlauf den Zustand des Implantats und beispielsweise dessen Lebensdauer beeinflussen.

Es ist deshalb bekannt, Sensoren in Testimplantaten, aber auch in längerfristig im Körper eingesetzten Implantaten vorzusehen, um dem medizinischen Personal die Möglichkeit zu geben, Informationen über den Heilungsverlauf zu erhalten.

In der US 2017/0196508 A1 wird das Vorsehen von Sensoren in oder an Spinalimplantaten beschrieben. Bei den Sensoren kann es sich insbesondere um Beschleunigungs- und/oder Dehnungsmesser handeln, die an verschiedenen Stellen des Pedikelsystems angeordnet sein können, um den Heilungsverlauf zu überwachen. Es wird u.a. die Verwendung von passiven MEMS (mikro-elektro-mechanisches System)-Sensoren vorgeschlagen.

Weitere Dokumente, welche von demselben Anmelder bzw. Inhaber wie die US 2017/0196508 A1 stammen und ebenfalls das Vorsehen von passiven MEMS-Sensoren in Implantaten offenbaren, sind die WO 2015/20070 A1, WO 2015/200723 A1, WO 2015/200707 A1, WO 2015/200722 A2, WO 2015/200720 A2, WO 2014/209916 A1, WO 2014/144107 A1, WO 2014/144070 A1, WO 2014/100795 A1 und die WO 2015/200718 A1.

MEMS-Sensoren sind aber verhältnismässig aufwändig in der Herstellung und benötigen zum drahtlosen Auslesen weiteren elektronische Bauteile.

In der US 9,629,583 B2 ist eine in einen Knochen einschraubbare Schraube offenbart, in deren Inneren ein Sensor, sowie Verarbeitungs-, Speicher- u. Energiespeichermittel angeordnet sind. Des Weiteren ist eine Übermittlungseinheit zur drahtlosen Übermittlung von Daten vorgesehen.

Die WO 2017/004483 A1 offenbart ein Pedikelsystem mit einem am Verbindungsstab angebrachten Sensor zur Messung von Kompression, Biegung und/oder Torsion des Verbindungsstabs. Ebenso wird ein intervertebraler Abstandshalter mit einem Sensor offenbart. Beim Sensor handelt es sich um einen Dehnungsmessstreifen oder einen piezoelektrischen Sensor. Beide diese Sensorarten sind relativ aufwändig in der Herstellung und entsprechend teuer. Ausserdem werden weitere elektronische Komponenten benötigt, um ein drahtloses Auslesen der Sensordaten zu ermöglichen.

Die WO 2007/090005 A1 beschäftigt sich mit Spinalimplantaten. Die Spinalimplantate werden zwischen zwei benachbarten Wirbelkörpern eingesetzt und weisen einen Sensor zum Messen von Lasten auf. Als Beispiel für einen Sensor wird ein Dehnungsmessstreifen angegeben.

Die WO 2007/098385 A2 offenbart einen Sensor, der zwischen jeweils zwei Wirbelkörpern implantierbar ist, um die dort auftretenden Lasten zu messen. Die Auslesung des Sensors kann drahtlos, zum Beispiel via RFID, erfolgen.

Die WO 2007/025191 A1 beschreibt das Anbringen von Sensoren an einem orthopädischen Knochenimplantat, wie insbesondere einem intramedullären Nagel, zum Messen von Beugung, Torsion und Kompression des Implantates. Die Sensoren sind in Vertiefungen eingesetzt, welche auf der Aussenseite des Implantates vorgesehen sind. Die Messdaten können drahtlos nach aussen übertragen werden.

In der Patentanmeldung WO 2017/116343 A1 wird ein Pedikelsystem mit einem am Verbindungsstab angeordneten Sensor zum Messen und Übermitteln von Kraft-, Deformations- und Verschiebungsdaten offenbart.

Ferner offenbaren die WO 2021/15485 A2, die EP 3 772 350 A1, die WO 2020/247890 A1, die WO 2017/165717 A1 sowie die US 11,042,916 B1 jeweils Implantate mit aktiven Sensoren, das heisst mit Sensoren, die zum Beispiel über eine im Implantat untergebrachte Batterie mit Strom versorgt werden, um Messungen durchzuführen und die Messdaten in einem Speicherchip zu speichern, der ebenfalls im Implantat untergebracht ist. Das Auslesen erfolgt dann üblicherweise über eine von der Batterie mit Strom versorgte Sendeeinheit.

Die Dokumente WO 2020/206373 A1 und WO 2016/044651 A1 schlagen zwar jeweils das Anordnen von passiven Sensoren in einem Implantat vor, führen jedoch nicht weiter aus, auf welchem Messprinzip diese Sensoren beruhen sollen.

Ferner beschreiben die beiden fachfremden Dokumente WO 2007/116218 A1 und EP 3 150 998 B1 jeweils die Messung von mechanischen Eigenschaften von Bauteilen mittels eines Mikrodrahtes und unter Ausnutzung des Barkhausen-Effekts.

Die WO 2020/035217 A1 schlägt die Verwendung eines Mikrodrahtes vor in medizinischen Geräten, die, wie zum Beispiel Insulinpumpen, zur Verabreichung eines Medikaments ausgebildet sind.

### DARSTELLUNG DER ERFINDUNG

Es ist eine Aufgabe der vorliegenden Erfindung, ein einfach herstellbares und zur Stabilisierung eines Knochenbereiches dienendes medizinisches Knochenimplantat mit einem aus einem thermoplastischen Kompositmaterial hergestellten Implantatkörper und mit zumindest einem Sensor anzugeben, welches ein drahtloses Auslesen von Sensormessdaten erlaubt. Zur Lösung dieser Aufgabe wird ein Knochenimplantat vorgeschlagen, wie es in Anspruch 1 angegeben ist. Eine weitere Aufgabe der vorliegenden Erfindung besteht darin, ein einfaches Verfahren anzugeben, das eine Überwachung des Zustands eines Implantats. Ein Verfahren, welches diese weitere Aufgabe löst, ist im Anspruch 12 angegeben. Weitere Ausführungsformen sind in den abhängigen Ansprüchen angegeben.

Die vorliegende Erfindung stellt also ein medizinisches Knochenimplantat zur Stabilisierung eines Knochenbereiches zur Verfügung, aufweisend
einen aus einem thermoplastischen Kompositmaterial hergestellten Implantatkörper zur Verankerung in oder an einem menschlichen oder tierischen Knochen zwecks Stabilisierung des Knochenbereiches; sowie
zumindest einen Sensor zur Messung von einem oder mehreren Parametern des Heilungsverlaufes und/oder eines Zustands des Implantats.

Bei dem zumindest einen Sensor handelt es sich um einen passiven magnetoelastischen Sensor.

Indem ein passiver magnetoelastischer Sensor verwendet wird, kann das Implantat besonders einfach und entsprechend kostengünstig hergestellt werden. Ein weiterer Vorteil besteht darin, dass ein passiver magnetoelastischer Sensor im Implantat in der Regel verhältnismässig wenig Platz beansprucht und deshalb die Implantateigenschaften nur minimal beeinflusst. Trotzdem ermöglicht ein passiver magnetoelastischer Sensor bei Bedarf ein drahtloses Auslesen der Sensordaten von ausserhalb des Körpers des Patienten. Es sind hierzu keine weiteren Elektronikbauteile, wie zum Beispiel ein elektrischer Schaltkreis oder eine separate Übermittlungseinheit notwendig. Insbesondere muss im Implantat auch kein Energiespeicher, wie eine Batterie, vorgesehen werden.

Wie bereits erwähnt, sind Knochenimplantate zumindest teilweise, meist vollständig, innerhalb des Körpers angeordnet und sind dabei in oder an einem Knochen verankert, das heisst daran befestigt. Die Verankerung des Implantats in oder an einem Knochen kann dabei auch indirekt, das heisst zum Beispiel via eine weitere künstlich hergestellte Komponente, insbesondere via ein weiteres Implantat mit bevorzugt ebenfalls einem aus einem thermoplastischen Kompositmaterial hergestellten Implantatkörper erfolgen. Dies ist in der Regel zum Beispiel bei Pedikelsystemen der Fall, wo ein erstes Knochenimplantat in Form eines Verbindungsstabes via eine Vielzahl von zweiten Knochenimplantaten in Form von Pedikelschrauben in den Wirbelkörpern verankert ist.

Ein Knochenimplantat dient dann zur Stabilisierung eines Knochenbereiches, wenn es einen Knochenbereich stützt und/oder festigt. Der Knochenbereich betrifft bevorzugt zumindest denjenigen Bereich eines Knochens, in oder an welchem der Implantatkörper verankert ist. Dabei kann der zu stabilisierende Knochenbereich lediglich einen oder auch mehrere Knochen oder Knochenteile betreffen. Insbesondere kann der Knochenbereich mehrere Wirbelkörper der Wirbelsäule betreffen. Der zu stabilisierende Knochenbereich kann aber zum Beispiel auch nur einen einzelnen Knochen betreffen, der beispielsweise nach einem Bruch mit Hilfe des Knochenimplantats zu stabilisieren ist, so dass die Knochenteile wieder wie gewünscht zusammenwachsen können. Beim Knochenimplantat kann es sich beispielsweise auch um einen Wirbelkörperersatz oder um einen Bandscheibenersatz handeln, da in der Regel auch diese an oder in zumindest einem Knochen verankert, d.h. daran befestigt, sind. Ein künstlicher Wirbelkörperersatz und ein künstlicher Bandscheibenersatz dienen dazu, einen Rückenwirbel bzw. eine Bandscheibe zu ersetzen und sind deshalb üblicherweise an oder in zumindest einem benachbarten Wirbelkörper, meist an oder in den zwei benachbarten Wirbelkörpern, verankert. Der Wirbelkörperersatz und der Bandscheibenersatz dienen dadurch jeweils dazu, die Wirbelsäule und insbesondere den Knochenbereich mit den beiden benachbarten Wirbelkörpern zu stabilisieren.

Der Implantatkörper bildet üblicherweise den Hauptbestandteil des Implantats, welcher dem Implantat bevorzugt seine strukturelle Festigkeit gibt. Das Implantat kann insbesondere ausschliesslich aus Implantatkörper und dem zumindest einen Sensor bestehen.

Der zumindest eine Sensor stellt ein Bauteil dar, das bestimmte Eigenschaften, insbesondere physikalische Eigenschaften, seiner Umgebung qualitativ oder als Messgrösse quantitativ erfassen kann. Die Umgebung wird dabei in der Regel durch den Implantatkörper und/oder den unmittelbaren Bereich des menschlichen bzw. tierischen Körpers um den Implantatkörper herum gebildet. Vor dem Implantieren des Implantats in den menschlichen bzw. tierischen Körper kann die Umgebung jedoch zum Beispiel auch durch ein Formpresswerkzeug (bei der Herstellung) oder durch Lagerungs- und Transportgebinde oder durch andere derartige Implantate (bei der Lagerung oder beim Transport) gebildet sein. Die Messgrösse wird dabei basierend auf dem magnetoelastischen Effekt erfasst und beim Auslesen in der Regel in ein elektrisches Signal umgeformt. Das Implantat kann einen einzigen magnetoelastischen Sensor oder eine Vielzahl magnetoelastischer Sensoren aufweisen.

Bei dem einen oder den mehreren Parametern des Heilungsverlaufes und/oder des Zustands des Implantats kann es sich insbesondere um eine mechanische Belastung, wie zum Beispiel eine Kompression, eine Streckung, eine Biegung oder eine Torsion, und/oder eine Temperatur des Implantats handeln. Der oder die gemessenen Parameter können alternativ oder zusätzlich aber zum Beispiel auch die Temperatur des menschlichen bzw. tierischen Körpers im unmittelbaren Bereich des Implantats betreffen. Grundsätzlich kann der Sensor zur Messung von jeglichen Parametern ausgebildet sein, die eine Änderung der magnetischen Eigenschaften des Sensormaterials bewirken können. Der oder die gemessenen Parameter ermöglichen somit Rückschlüsse über den Zustand des Implantats, das heisst es können Informationen z.B. über Druck, Zug und/oder Torsionskräfte, die auf dem Implantat lasten, erhalten werden. Es können insbesondere auch Rückschlüsse über die Stabilität des Implantatbereichs, also des Bereichs unmittelbar um das Implantat herum, erhalten werden. Der Implantatbereich kann dabei insbesondere weitere mit dem Implantat verbundene Implantate und/oder Körperstrukturen, wie beispielsweise Knochen, betreffen. Bei mehreren Messungen, die zu unterschiedlichen Zeitpunkten durchgeführt werden, können auch der zeitliche Verlauf dieser Parameter und somit allfällige Veränderungen des Implantatzustands evaluiert werden. Von den gemessenen Parametern bzw. dem Zustand des Implantats und insbesondere von deren Verlauf kann wiederum auf den Heilungsverlauf geschlossen werden. Von einer Messung der Temperatur kann beispielsweise auf eine Infektion im unmittelbaren Bereich um das Implantat herum geschlossen werden.

Die Messung basiert insbesondere auf einer Änderung der magnetischen Eigenschaften eines Materials des Sensors. Insbesondere wird durch den Einfluss einer auf das Implantat lastenden Kraft die magnetische Permeabilität des Sensormaterials verändert. Eine Veränderung der magnetischen Permeabilität des Sensormaterials kann auch durch eine Temperaturänderung bewirkt werden. Das Sensormaterial weist dabei bevorzugt eine Vormagnetisierung auf. Aufgrund des magnetoelastischen Effekts, der auch unter dem Namen "Villari-Effekt" bekannt ist, bewirkt eine am Sensormaterial angelegte Last und/oder Temperatur eine Änderung des Magnetfelds des Sensormaterials, welche messbar ist. Die Messung kann dabei insbesondere drahtlos erfolgen, was besonders vorteilhaft ist, da dadurch berührungslose Messungen von ausserhalb des Körpers ermöglicht werden.

Um eine Messung basierend auf dem magnetoelastischen Effekt zu ermöglichen, weist der zumindest eine Sensor in der Regel ein Sensormaterial in Form eines ferromagnetischen Materials, bevorzugt einer ferromagnetischen Legierung, mit einer inversen Magnetostriktion auf. Das ferromagnetische Material ändert unter mechanischer bzw. thermischer Belastung seine magnetischen Eigenschaften und insbesondere seine magnetische Permeabilität, was mit Hilfe eines geeigneten Messinstruments bzw. Auslesegeräts erfass- und messbar ist.

Es hat sich herausgestellt, dass besonders genaue Messungen dann möglich sind, wenn der zumindest eine Sensor zur Messung des einen oder der mehreren Parameter den Barkhausen-Effekt ausnutzt. Aufgrund des Barkhausen-Effekts bewirkt ein sich stetig änderndes Magnetfeld eine diskontinuierliche Änderung der Magnetisierung von ferromagnetischen Materialen. Der Grund hierfür liegt in der Anwesenheit von Bereichen einheitlicher Magnetisierungsrichtung, den sogenannten Weiss-Bezirken, die von Bloch-Wänden getrennt werden. Bei z.B. einer langsamen Erhöhung der magnetischen Feldstärke kippen deshalb die magnetischen Momente ganzer Bereiche, also der Weiss-Bezirke, auf einmal um und führen so zu einer sprunghaften Veränderung des Magnetfeldes des entsprechenden Materials. Aufgrund der bevorzugten Ausnutzung des Barkhausen-Effekts durch den zumindest einen Sensor, sind diese sprunghaften Magnetfeldveränderungen messbar.

Eine besonders sensitive und genaue Messung ist möglich, wenn es sich bei dem zumindest einen Sensor um einen Mikrodraht handelt. Die üblichen geometrischen Abmessungen eines Mikrodrahtes können bei der Messung ein besonders ausgeprägtes Signal aufgrund des magnetoelastischen Effekts bewirken. Das Signal ist bei einem Mikrodraht zudem insbesondere dann besonders ausgeprägt, wenn zur Messung der Barkhausen-Effekt ausgenutzt wird. Ein Mikrodraht kann zudem besonders vorteilhaft im Inneren eines Implantats angeordnet werden, zum Beispiel so, dass er sich entlang von dessen Hauptlängsmittellinie erstreckt.

Der Mikrodraht hat bevorzugt einen Durchmesser von 10 µm bis 250 µm. Die Länge des Mikrodrahtes ist bevorzugt mehr als 25 mal, bevorzugter mehr als 50 mal, am bevorzugtesten mehr als 100 mal, grösser als der Durchmesser.

In einer bevorzugten Ausführungsform ist der zumindest eine Sensor zumindest teilweise, noch bevorzugter vollständig, im Inneren des Implantatkörpers angeordnet. Der Sensor ist dadurch optimal vor äusseren Einflüssen geschützt und kann Messwerte direkt aus dem Inneren des Implantats liefern. Umgekehrt ist dadurch aber auch das umliegende Körpergewebe vom Sensor getrennt, so dass es zu keiner Unverträglichkeit mit dem Sensormaterial kommen kann. Um optimale Messresultate zu erreichen, kann der zumindest eine Sensor insbesondere im Material des Implantatkörpers eingebettet sein.

In einer insbesondere bevorzugten Ausführungsform ist der Implantatkörper aus einem faserverstärkten Kunststoff, hergestellt. Beim faserverstärkten Kunststoff handelt es sich bevorzugt um einen kohlefaserverstärkten Kunststoff, wie insbesondere Polyetheretherketon (PEEK). Die Herstellung des Implantatkörpers aus einem thermoplastischen Kunststoffmaterial und insbesondere aus einem faserverstärkten Kunststoff hat einerseits den Vorteil, dass dadurch strukturell sehr feste Implantate herstellbar sind, die zudem biokompatibel und für bildgebende Verfahren, wie z.B. MRI, vorteilhaft sind. Andererseits ist thermoplastisches Kunststoffmaterial und insbesondere faserverstärkter Kunststoff, im Gegensatz etwa zu Metallen, aber auch durchlässig für elektromagnetische Strahlung. Das Vornehmen von Sensormessungen ist deshalb auch dann problemlos möglich, wenn sich der Sensor vollständig im Inneren des Implantatkörpers befindet.

Falls der Implantatkörper aus einem faserverstärkten Kunststoff hergestellt ist, beträgt die Faserlänge vorzugsweise mindestens 1 mm, bevorzugter mindestens 5 mm. Insbesondere bevorzugt ist die Länge der Fasern jedoch so gross, dass sie der kompletten Länge der Haupterstreckungsrichtung des Implantatkörpers entspricht. Der Implantatkörper erhält dadurch eine besonders hohe Steifigkeit und strukturelle Festigkeit.

Eine gute Implantatfestigkeit wird erreicht, wenn das Faservolumengehalt in einem Bereich von 20 bis 80 %, bevorzugt in einem Bereich von 35 - 70 %, besonders bevorzugt in einem Bereich von 45 - 60 %, liegt.

Als Kunststoffe werden bevorzugt Thermoplaste verwendet, besonders bevorzugt sogenannte Hochtemperaturthermoplaste aus den Familien der Polyaryletherketone, Polyimide und/oder Polysulfone.

Insbesondere bevorzugt ist eine Ausführungsform, bei der das Implantat eine Hauptlängsmittellinie aufweist und sich der zumindest eine Sensor im Wesentlichen entlang der gesamten Längserstreckung dieser Hauptlängsmittellinie erstreckt. Die Hauptlängsmittellinie stellt eine Linie dar, welche sich entlang ihrer gesamten Längserstreckung zentral innerhalb des Implantats, insbesondere innerhalb des Implantatkörpers, erstreckt. Der zumindest eine Sensor ist somit bevorzugt im Wesentlichen gleich lang wie das Implantat. Wenn das Implantat insgesamt z.B. eine gebogene oder eine spiralförmige Gestalt beschreibt, weist auch die Hauptlängsmittellinie eine entsprechend gebogene bzw. spiralförmige Gestalt auf, so dass der zumindest eine Sensor als Ganzes ebenfalls eine gebogene bzw. spiralförmige Gestalt hat. Dies hat den Vorteil, dass Messungen entlang der gesamten Längserstreckung des Implantats möglich sind, so dass die Messung das gesamte Implantat erfassen kann und nicht auf einzelne Stellen des Implantats beschränkt ist. Das Implantat kann dadurch somit über seine ganze Länge vollständig kontrolliert und überwacht werden.

Wenn es sich beim zumindest einen Sensor um einen Mikrodraht handelt, kann sich dieser entweder komplett durchgehend oder gestückelt in aneinander gereihten Abschnitten im Wesentlichen entlang der gesamten Längserstreckung dieser Hauptlängsmittellinie erstrecken. Mit anderen Worten kann das Implantat mehrere Sensoren, das heisst Mikrodrähte, aufweisen, welche in regelmässigen Abständen entlang der Hauptlängsmittellinie angeordnet sind. Falls mehrere aneinander gereihte Abschnitte vorgesehen sind, sind diese vorteilhaft in regelmässigen Abständen zueinander angeordnet. Ein Vorteil von mehreren aneinander gereihten Abschnitten kann sein, dass separate Messungen für die verschiedenen Abschnitte möglich sind, so dass z.B. Unterschiede in der Lastverteilung entlang der Hauptlängsmittellinie des Implantatkörpers messbar werden.

Das Implantat kann auch mehrere derartige Sensoren aufweisen, welche sich parallel zueinander erstrecken. Vorzugsweise erstrecken sich die Sensoren dabei nicht nur parallel zueinander, sondern auch parallel zur Hauptlängsmittellinie, welche je nach Ausführung des Implantats gebogen sein kann. Indem mehrere parallel zueinander angeordnete Sensoren vorgesehen sind, wird es insbesondere möglich, auch Torsionskräfte zu erfassen, die auf das Implantat wirken.

Beim Implantat kann es sich insbesondere um ein Wirbelsäulenimplantat, wie einen künstlichen Wirbelkörperersatz, einen künstlichen Bandscheibenersatz, einen Dübel, einen Schrauben-Anker, eine Fixierungsplatte, eine Pedikelschraube oder einen Verbindungsstab eines Pedikelsystems handeln. Im Falle einer Fixierungsplatte kann es sich um eine Platte zur Fixierung der Wirbelsäule in einem anterioren, lateralen oder posterioren Bereich handeln. Alternativ kann es sich beim Implantat auch um ein Traumaimplantat, wie insbesondere ein generelles Ankersystem, das heisst zum Beispiel einen Schrauben-Anker oder einen Dübel, oder eine Knochenplatte oder eine Knochenschraube, handeln.

Der zumindest eine Sensor hat bevorzugt eine Temperaturwiderstandsfähigkeit bis mindestens 450 °C. Das heisst, der zumindest eine Sensor wird nicht beschädigt, solange er einer Temperatur ausgesetzt wird, die nicht grösser als 450 °C ist. Vorteilhaft sind mittels des zumindest einen Sensors bis zu einer Temperatur von 450 °C auch Messungen möglich. Eine derartige Ausbildung des zumindest einen Sensors hat Vorteile bzgl. der Implantatherstellung. Der zumindest eine Sensor kann dadurch bereits früh im Herstellungsprozess im Implantat integriert werden, ohne dass er durch die erhöhten Temperaturwerte beschädigt wird. Vorteilhaft ist es dadurch sogar möglich, bereits während der Herstellung Messungen mit Hilfe des zumindest einen Sensors durchzuführen, um den Herstellungsprozess zu überwachen. Beispielsweise können während der Herstellung auf den Implantatkörper einwirkende Einflüsse, wie insbesondere Druck und Temperatur, gemessen werden, um dadurch Rückschlüsse auf die Implantatsqualität zu erhalten.

Die vorliegende Erfindung bezieht sich ausserdem auf ein Verfahren zur Überwachung eines Zustands eines Implantats und/oder des Heilungsverlaufs eines menschlichen oder tierischen Körpers, in welchen das Implantat eingesetzt ist, wobei es sich beim Implantat insbesondere um ein Knochenimplantat wie oben angegeben handeln kann, und wobei das Implantat zumindest einen passiven magnetoelastischen Sensor zur Messung von einem oder mehreren Parametern des Heilungsverlaufes und/oder des Zustands des Implantats aufweist. Der Sensor wird dabei zur Überwachung des Zustands des Implantats während dessen Herstellung und/oder während dessen Lagerung verwendet.

Bevorzugt werden während der Herstellung und/oder Lagerung des Implantats insbesondere die Verläufe von Druck und Temperatur gemessen und vorteilhaft auch aufgezeichnet. Auf diese Weise können die Integrität und die Unversehrtheit des Implantats sichergestellt werden. Implantate, bei denen Druck und/oder Temperatur bei der Herstellung und/oder Lagerung zum Beispiel nicht innerhalb bestimmter Grenzwerte verlaufen, können ausgeschieden werden. Auf diese Weise kann verhindert werden, dass Implantate in den menschlichen oder tierischen Körper implantiert werden, die Herstellungsfehler und/oder Lagerschäden aufweisen. Eine derartige Überwachung des Implantats mit Hilfe des zumindest einen Sensors bereits während der Herstellung und/oder Lagerung ist nicht nur mit Knochenimplantaten möglich, sondern auch mit beliebigen anderen Implantaten.

Bevorzugt wird der Zustand des Implantats nicht nur während der Herstellung und/oder Lagerung mittels des zumindest einen Sensors kontrolliert, sondern auch unmittelbar nach dessen Implantierung in einem menschlichen oder tierischen Körper. Mit "unmittelbar nach dessen Implantierung" ist gemeint, dass z.B. die auf das Implantat einwirkenden Kräfte direkt nach dem Einsetzen des Implantats in den Körper gemessen werden, also ganz zu Beginn des Heilungsverlaufes. Auf diese Weise kann insbesondere die korrekte Positionierung des Implantats im Körper geprüft werden.

Alternativ oder zusätzlich, aber bevorzugt, wird ausserdem der Heilungsverlauf nach der Implantierung des Implantats in einem menschlichen oder tierischen Körper mittels des zumindest einen Sensors überwacht. Die Überwachung, also z.B. die Messung der auf das Implantat wirkenden Kräfte und/oder die Messung der Temperatur mit Hilfe des zumindest einen Sensors, kann insbesondere in regelmässigen zeitlichen Abständen durchgeführt werden. Mit Hilfe des Verlaufs von z.B. den auf das Implantat wirkenden Kräfte kann auf den Heilungsverlauf geschlossen werden und in Abhängigkeit davon die Behandlung zum Beispiel beendet oder angepasst werden.

Zur Herstellung des Implantats werden bevorzugt eine Vielzahl von Prepregs, insbesondere von unidirektionalen Prepregs, verwendet, die unter Druck und Hitze miteinander verpresst werden. Unter Prepregs werden Faser-Matrix-Halbzeuge verstanden, also Halbzeuge mit Verstärkungsfasern, die bevorzugt in einer Kunststoffmatrix angeordnet sind. Bei den Verstärkungsfasern handelt es sich bevorzugt um Kohlefasern. Die Prepregs können z.B. in Strang-, Band- oder Plattenform vorliegen. Durch das Verpressen der Prepregs unter Druck und Hitze werden die Prepregs bevorzugt miteinander verschweisst und in die endgültige Form des Implantats gebracht. Die Prepregs bilden nach dem Verpressen bevorzugt den Implantatkörper.

Der zumindest eine Sensor kann vor dem Verpressen zwischen den Prepregs angeordnet werden, welche dann so miteinander verpresst werden, dass der Sensor auch während dem Verpressen zwischen den Prepregs angeordnet ist und dadurch im fertigen Implantat zumindest teilweise, bevorzugt vollständig, im Inneren des Implantatkörpers zu liegen kommt. Alternativ kann der zumindest eine Sensor beim Verpressen auch bereits in einem der Prepregs eingebettet sein. Das Einbetten des Sensors in einen der Prepregs erfolgt dann also bereits vor dem Verpressen. Auf diese Weise kann der Sensor im Implantat optimal positioniert werden.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Fig. 1: eine schematische Ansicht eines in den Wirbelkörpern einer Wirbelsäule verankerten Knochenimplantats in Form eines Pedikelfixationssystems gemäss einer ersten erfindungsgemässen Ausführungsform;
- Fig. 2a: eine Seitenansicht des Verbindungsstabes des Pedikelfixationssystems der Fig. 1;
- Fig. 2b: eine Querschnittsansicht durch den Verbindungsstab der Fig. 2a in der Ebene II-II;
- Fig. 3a: eine Seitenansicht eines Knochenimplantats in Form eines Verbindungsstabes eines Pedikelfixationssystems gemäss einer zweiten erfindungsgemässen Ausführungsform,
- Fig. 3b: eine Querschnittsansicht durch den Verbindungsstab der Fig. 3a in der Ebene III-III;
- Fig. 4a: eine Seitenansicht eines Knochenimplantats in Form eines Verbindungsstabes eines Pedikelfixationssystems gemäss einer dritten erfindungsgemässen Ausführungsform,
- Fig. 4b: eine Querschnittsansicht durch den Verbindungsstab der Fig. 4a in der Ebene IV-IV;
- Fig. 5: ein Flussdiagramm eines Verfahrens zur Herstellung eines erfindungsgemässen Knochenimplantats gemäss einer ersten Variante;
- Fig. 6: ein Flussdiagramm eines Verfahrens zur Herstellung eines erfindungsgemässen Knochenimplantats gemäss einer zweiten Variante;
- Fig. 7a: eine schematische Querschnittsansicht von mehreren in ein Pressformwerkzeug eingelegten Prepregs, unmittelbar vor dem Verpressen zwecks Herstellung eines anderen erfindungsgemässen Knochenimplantats;
- Fig. 7b: eine schematische Querschnittsansicht des Pressformwerkzeugs der Fig. 7a, beim Verpressen der Prepregs;
- Fig. 8: eine schematische Ansicht des Herstellungsschritts der Fig. 7b, beim Auslesen des Sensors zur Überwachung des Pressvorgangs; sowie
- Fig. 9: eine schematische Ansicht des Knochenimplantats der Fig. 1, beim Auslesen des Sensors zur Überwachung des Heilungsverlaufs.

### BESCHREIBUNG BEVORZUGTER AUSFÜHRUNGSFORMEN

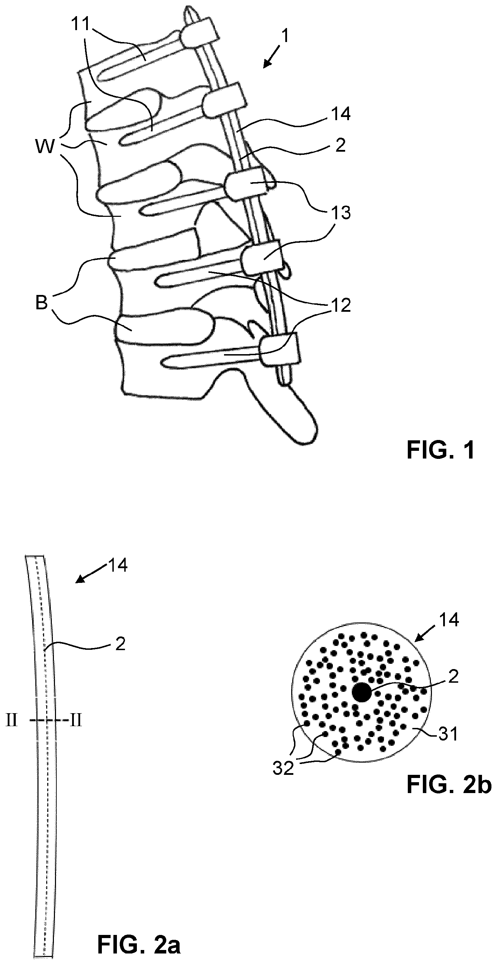

In den Figuren 1 bis 9 sind verschiedene erfindungsgemässe Ausführungsformen von medizinischen Knochenimplantaten sowie unterschiedliche bevorzugte Herstellungsarten von derartigen Implantaten gezeigt. Gleich oder ähnlich wirkende Elemente der verschiedenen Ausführungsformen sind im Folgenden jeweils mit denselben Bezugszeichen versehen.

Fig. 1 stellt ein dynamisches Pedikelfixationssystem 1 dar, das in mehreren Wirbeln W der Wirbelsäule eines Patienten verankert ist. Die Wirbel W sind durch Bandscheiben B voneinander getrennt. Das Pedikelfixationssystem 1, welches zur Stabilisierung des gezeigten Knochenbereiches dient, bildet als Ganzes ein Knochenimplantat und umfasst mehrere Pedikelschrauben 11, die mit jeweils einem Schraubenschaft 12 in den Pedikel eines Wirbels W eingeschraubt sind. Jede Schraube ist via einen vorteilhaft polyaxial einstellbaren Tulpenkopf 13 an einem durchgehenden Verbindungsstab 14 befestigt. Die Pedikelschrauben 11, die Tulpenköpfe 13 und der Verbindungsstab 14 bilden aufgrund ihrer Anordnung im Inneren des Körpers und ihrer (direkten oder indirekten) Befestigung an der Wirbelsäule jeweils selbst ein Knochenimplantat.

Andere Ausführungsformen des in der Fig. 1 gezeigten Fixationssystems sind ebenfalls denkbar. So kann die Anzahl der Pedikelschrauben 11 dahingehend variiert werden, dass zum Beispiel anstatt der fünf gezeigten Pedikelschrauben nur zwei Pedikelschrauben Verwendung finden. Ebenso kann die Anzahl der Pedikelschrauben auch höher als fünf sein. Des Weiteren kann das Pedikelsystem, wie es üblicherweise der Fall ist, zwei parallele Reihen von Pedikelschrauben aufweisen, die mit jeweils einem Verbindungsstab verbunden sind.

Im Inneren des leicht gebogenen Verbindungsstabes 14 sind ein oder mehrere Sensoren angeordnet. Im vorliegenden Fall wird der Sensor von einem Mikrodraht 2 gebildet, der sich in Längsrichtung durchgehend entlang der Hauptlängsmittellinie des Verbindungsstabes 14 erstreckt. Passive Sensoren, z.B. ebenfalls in Form eines Mikrodrahtes, können auch in den jeweiligen Pedikelschrauben 11 eingebettet sein.

Der Mikrodraht 2 ist aus einem ferromagnetischen Material hergestellt, das eine inverse Magnetostriktion aufweist. Bei einer Veränderung der auf den Verbindungsstab 14 und somit den Mikrodraht 2 wirkenden Kräfte und/oder Temperatur, wird der Mikrodraht 2 z.B. geringfügig verformt, gestaucht, verdreht und/oder gedehnt. Aufgrund des magnetoelastischen Effekts verändert sich dadurch die magnetische Permeabilität des Materials des Mikrodrahtes 2, was mit Hilfe eines Auslesegeräts 5 (siehe Fig. 11) detektier- und messbar ist.

Die Fig. 2a zeigt den Verbindungsstab 14 des Pedikelfixationssystems der Fig. 1 in Alleinstellung von der Seite her. Die leichte Krümmung des Verbindungsstabes 14 ist erkennbar. Die gestrichelte Linie zeigt die Lage des Mikrodrahtes 2, welcher sich direkt auf der Hauptlängsmittellinie des Implantats und über dessen gesamte Länge erstreckt.

In der Querschnittsansicht der Fig. 2b ist schematisch die innere Struktur desselben Verbindungsstabes 14 gezeigt. Der Implantatkörper wird durch eine Kunststoffmatrix 31 gebildet, in welcher eine Vielzahl von Kohlefasern 32 eingebettet ist. Die Kohlefasern 32 erstrecken sich parallel zueinander entlang der Hauptlängsmittellinie des Verbindungsstabes 14. Der Verbindungsstab 14 erhält dadurch eine besonders gute strukturelle Festigkeit. Zentral in der Mitte des Verbindungsstabes 14 verläuft der Mikrodraht 2.

Als Kunststoffmatrix 31 werden Thermoplaste verwendet, bevorzugt sogenannte Hochtemperaturthermoplaste aus den Familien der Polyaryletherketone, Polyimide oder Polysulfone. Die Faserlänge der Kohlefasern beträgt mindestens 1 mm, jedoch bevorzugt eine Länge, die der kompletten Länge der Haupterstreckungsrichtung des Verbindungsstabes 14 entspricht.

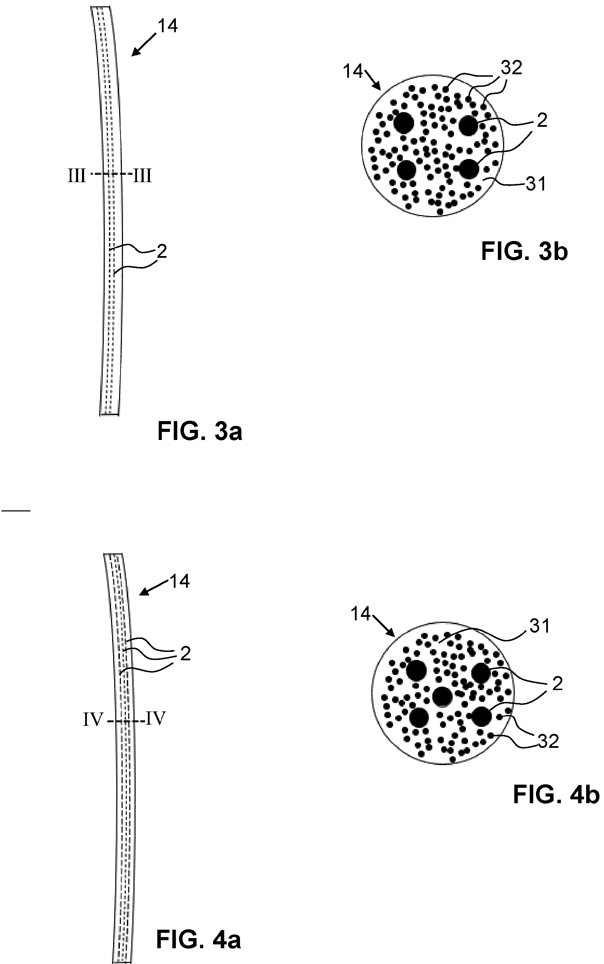

Der Faservolumengehalt ist in einem Bereich von 20 bis 80 %, bevorzugt zwischen einem Bereich von 35 - 70 %, besonders bevorzugt zwischen einem Bereich von 45 - 60 %. Der in der Fig. 4b gezeigte Verbindungsstab 14 weist einen kreisrunden Querschnitt auf. Andere Querschnittsformen, wie zum Beispiel vier- oder sechseckig, sind ebenfalls denkbar.

Der Durchmesser dieses in den Figs. 2a und 2b dargestellten Verbindungsstabes 14 ist bevorzugt in einem Bereich von 2 - 10 mm, bevorzugter in einem Bereich von 4 - 6 mm. Die geometrische Ausführung in Längsrichtung des Verbindungsstabes 14 kann gerade oder gekrümmt oder eine Kombination von beiden dahingehend sein, dass eine Sektion der Längsrichtung gerade ist und die angeschlossene Sektion gekrümmt ist. Ebenso ist eine geometrische Ausführung in Längsrichtung denkbar, bei der eine Sektion der Längsrichtung konvex und die angeschlossene Sektion konkav gekrümmt ist. Der Krümmungsradius der gekrümmten Ausführungen ist bevorzugt in einem Bereich von 50 - 600 mm. Andere Krümmungsradien sind ebenfalls denkbar. Die Länge des Verbindungsstabes 14 ist bevorzugt in einem Bereich von 50 - 500 mm. Andere Längen sind ebenfalls denkbar.

Der im Verbindungsstab 14 der Figs. 2a und 2b verwendete Sensor 2 ist ein passiver Sensor, welcher insbesondere unter thermischen Belastungen seine charakteristischen Materialeigenschaften ändert, die dann drahtlos erfasst und vom Körperinneren nach ausserhalb des Körpers übertragen werden können. Bevorzugt wird hierzu ein magnetoelastischer Mikrodraht verwendet, welcher eine ferromagnetische Legierung sowie eine inverse Magnetostriktion aufweist, so dass der Mikrodraht 2 unter thermischer Belastung seine magnetischen Eigenschaften ändert. Die Änderung der magnetischen Eigenschaften kann insbesondere in der Änderung der magnetischen Permeabilität erfasst und gemessen werden. Die bekannten physikalischen Phänomene hierzu sind vor allem der Villari-Effekt und bevorzugt der Barkhausen-Sprung. Dem Fachmann ist bekannt, wie der zumindest eine Sensor, das heisst hier der Mikrodraht 2, beschaffen sein muss und wie die Auslesung erfolgen muss, um die Messung auf dem Barkhausen-Effekt zu basieren. Hinweise hierzu erhält der Fachmann beispielsweise aus der WO 2007/116218 A1 und der EP 3 150 998 B1.

Dabei werden Ausführungen des Mikrodrahtes 2 bevorzugt angewendet, die einen Durchmesser im Bereich von 10 µm bis 250 µm haben und sich vorteilhaft über die ganze Implantatlänge erstrecken, entweder komplett durchgehend oder gestückelt in aneinander gereihten Abschnitten und damit auch die ganze Bauteillänge des Verbindungsstabes 14 vollständig kontrolliert und überwacht werden kann. Eine besonders bevorzugte Ausführung solcher passiven Sensoren sind jene, welche eine Temperaturwiderstandsfähigkeit von bis mind. 450 °C haben und eine Genauigkeit in der Temperaturerfassung von mindestens ± 0.1 K besitzen. Die bevorzugte Positionierung des Mikrodrahtes 2 im Verbindungsstab 14 ist mittig, jedoch ist auch eine aussermittige Positionierung denkbar. Mit dieser Ausführung dieses Mikrodrahtes 2 im Verbindungsstab 14 lassen sich vorgängig zur Implantierung auch die Temperaturen während der Herstellung und/oder Lagerung des Verbindungsstabes 14 aufzeichnen und drahtlos übermitteln.

Die Anzahl an Sensoren in der Ausführung der Fig. 2b ist eins. Jedoch können auch mehrere Sensoren der gleichen Art im Verbindungsstab 14 integriert sein.

Die in den Figs. 3a und 3b gezeigte Ausführungsform unterscheidet sich von derjenigen der Figs. 2a und 2b dadurch, dass im Verbindungsstab 14 mehrere, konkret vier, Mikrodrähte 2 vorgesehen sind, die parallel zueinander entlang der Hauptlängsmittellinie des Verbindungsstabes 14 verlaufen. Alle vier Mikrodrähte 2 sind jedoch beabstandet zur Hauptlängsmittellinie angeordnet, verlaufen also dezentral im Verbindungsstab 14. Die Mikrodrähte 2 dienen hier insbesondere zur Messung von mechanischen Belastungen, z.B. Zug, Druck, Biegung und/oder Torsion. Selbstverständlich können in anderen Ausführungsformen auch mehr als vier oder weniger als vier Mikrodrähte 2 im Verbindungsstab 14 integriert sein. Ebenso ist auch eine mittige Positionierung eines Mikrodrahtes 2 denkbar, insbesondere wenn von diesem Zug- und Druckkräfte aufgenommen und gemessen werden sollen.

Somit sind bei einer mittigen Positionierung des Mikrodrahtes 2, wie es zum Beispiel in der Ausführungsform der Figuren 2a und 2b der Fall ist, Zug- und Druckkräfte messbar. Bei einer dezentralen Positionierung des Mikrodrahtes 2 hingegen, wie es zum Beispiel in der Ausführungsform der Figuren 3a und 3b der Fall ist, können zusätzlich oder stattdessen Biege- und Torsionskräfte erfasst werden.

Die in den Figs. 4a und 4b gezeigte Ausführungsform unterscheidet sich von derjenigen der Figs. 2a, 2b und 3a, 3b dadurch, dass hier fünf Mikrodrähte 2 vorgesehen sind, die sich parallel zueinander innerhalb des Verbindungsstabes 14 erstrecken, wobei einer davon ungefähr mittig im Verbindungsstab 14 angeordnet ist und die anderen beabstandet dazu. Die vier aussermittig angeordneten Mikrodrähte 2 dienen zur Messung von mechanischen Belastungen, wie z.B. Zug, Druck, Biegung und/oder Torsion. Der ungefähr mittig angeordnete Mikrodraht 2 dient hingegen zur Messung von thermischen Belastungen. Selbstverständlich sind auch hier andere Anordnungen und eine andere Anzahl von Mikrodrähten 2 möglich.

Die in den Ausführungsformen der Figs. 2a bis 4b vorgesehenen passiven Sensoren müssen nicht zwingend als Mikrodrähte ausgebildet sein, sondern können auch andere Formen haben.

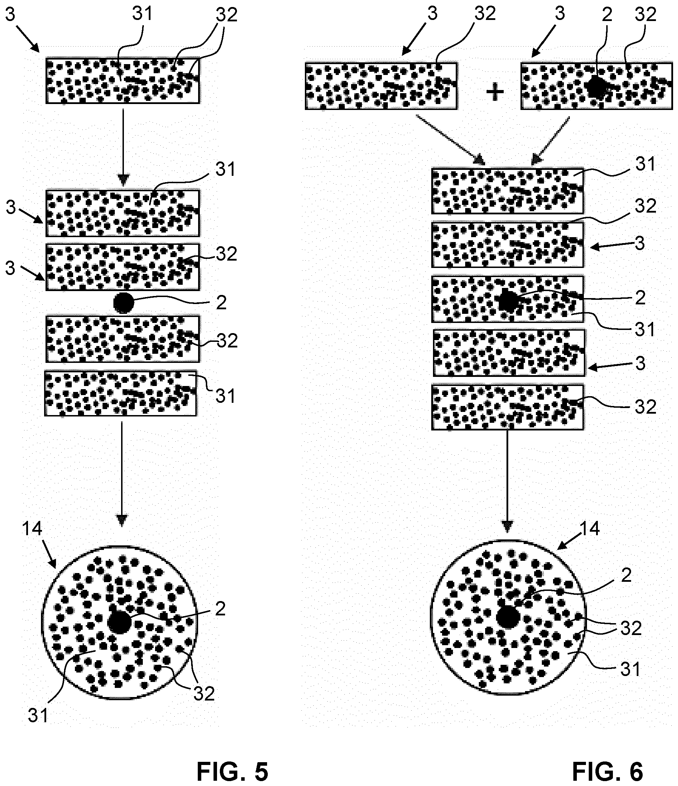

Die Figs. 5 und 6 stellen jeweils ein Flussdiagramm dar, das die Herstellung eines erfindungsgemässen Implantates vom Rohmaterial bis zum finalen Bauteil visualisiert. Als Beispiel dient hier die Herstellung des in den Figs. 2a und 2b gezeigten Verbindungsstabes 14. Dabei gibt es zur Herstellung des Verbindungsstabes 14 zwei Varianten, welche gleichermassen bevorzugt sein können:
In der ersten Variante, welche in der Fig. 5 gezeigt ist, werden in diesem Beispiel als Ausgangsmaterialien unidirektional faserverstärkte Prepregs 3 verwendet, welche jeweils Kohlefasern 32 aufweisen, die in einer Kunststoffmatrix 31 eingebettet sind. Ebenso können auch Gewebeprepregs verwendet werden. Die in dieser ersten Variante verwendeten unidirektional faserverstärkten Prepregs 3 sind jeweils geometrisch flächig mit einer bevorzugten Breite von 1 - 50 mm und einer bevorzugten Dicke von 0.1 - 0.5 mm. Andere Abmessungen hinsichtlich Breite und Dicke sind ebenfalls möglich. Andere geometrische Ausführungen, zum Beispiel kreisrund, sind ebenfalls möglich. Die Prepregs 3 werden in der Regel in der Länge zugeschnitten, wobei die Länge bevorzugt mindestens der Länge des Verbindungsstabes 14 entspricht. Als Fasern werden bevorzugt Kohlenstofffasern 32 verwendet, welche eine Faserlänge von mindestens 1 mm, jedoch bevorzugt eine Länge haben, welche der kompletten Länge der Haupterstreckungsrichtung des Verbindungsstabes 14 entsprechen.

Der Faservolumengehalt ist in einem Bereich von 20 bis 80 %, bevorzugt in einem Bereich von 35 - 70 %, besonders bevorzugt in einem Bereich von 45 - 60 %. Als Material für die Kunststoffmatrix 31 werden Thermoplaste verwendet, bevorzugt sogenannte Hochtemperaturthermoplaste aus den Familien der Polyaryletherketone, Polyimide und Polysulfone.

Die Prepregs 3 werden in einem zweiten Schritt zu Vorformlingen geometrisch exakt angeordnet bzw. gestapelt und dazwischen wird der Mikrodraht 2 mittig positioniert. Die Anordnung der Prepregs 3 kann manuell oder automatisch, zum Beispiel mit einem Tapelegeprozess oder mit einem 3D-Drucker, erfolgen. Die Positionierung des Sensors, also hier des Mikrodrahtes 2, kann manuell oder automatisch, zum Beispiel mit einem 3D-Drucker, erfolgen. Die Genauigkeit der Anordnung der Prepregs 3 und des Sensors kann mit Hilfe von Druck und Temperatur dahingehend verbessert werden, dass die Prepregs 3 untereinander und mit dem Mikrodraht 2 vorverschweisst und danach abgekühlt werden.

In einem dritten Schritt wird dann der Vorformling zum Beispiel in einem Presswerkzeug auf eine Verarbeitungstemperatur oberhalb des Schmelzpunktes der Kunststoffmatrix 31 erhitzt und auf die Endkontur des Verbindungsstabes 14 verpresst und abgekühlt, wobei hier der integrierte Mikrodraht 2 bereits die Herstelldaten bezüglich des Druckes und der Temperatur des Verbindungsstabes 14 zwecks Aufzeichnung drahtlos übermitteln kann.

In der zweiten Variante, welche in der Fig. 6 dargestellt ist, werden in diesem Beispiel als Ausgangsmaterialien zwei unterschiedliche Typen von unidirektional faserverstärkten Prepregs 3 verwendet. Die Zusammensetzung des einen Typs von Prepregs 3 ist analog zu derjenigen der in der ersten Variante verwendeten Prepregs 3. Bei der vorliegenden zweiten Variante wird jedoch zumindest ein Prepreg 3 verwendet, der zwar die gleiche Zusammensetzung hat wie die anderen Prepregs 3, jedoch zusätzlich einen mittig eingebetteten Sensor in Form eines Mikrodrahtes 2 enthält. Die Längen, Dicken und Geometrien der Prepregs entsprechen denjenigen der ersten Variante. Die Länge des Mikrodrahtes 2 entspricht mindestens der kompletten Länge des Verbindungsstabes 14.

Die Prepregs 3 werden auch bei dieser zweiten Variante in einem zweiten Schritt zu Vorformlingen geometrisch exakt angeordnet bzw. gestapelt, wobei nun aber der Prepreg 3 mit dem Mikrodraht 2 dazwischen mittig positioniert wird. Die Anordnung der Prepregs 3 kann manuell oder automatisch, zum Beispiel mit einem Tapelegeprozess oder mit einem 3D-Drucker, erfolgen.

In einem dritten Schritt wird auch hier dann der Vorformling zum Beispiel in einem Presswerkzeug auf eine Verarbeitungstemperatur oberhalb des Schmelzpunktes des Kunststoffmatrix 31 bzw. des Prepregs 3 erhitzt und auf die Endkontur des Verbindungsstabes 14 verpresst und abgekühlt, wobei auch hier der integrierte Mikrodraht 2 bereits die Herstelldaten bezüglich des Druckes und der Temperatur des Verbindungsstabes 14 zwecks Aufzeichnung drahtlos übermitteln kann.

Die in den Figs. 5 und 6 gezeigten und beschriebenen Varianten zur Herstellung des Verbindungsstabes 14 mit integrierten Sensoren sind vorzugsweise endkonturnahe Herstellungsverfahren, d.h. dass solch hergestellte Implantate hinsichtlich Form und Abmessung in der Regel nur eine geringfügige Nachbearbeitung erfordern, bei der die Positionierung der Sensoren beibehalten wird.

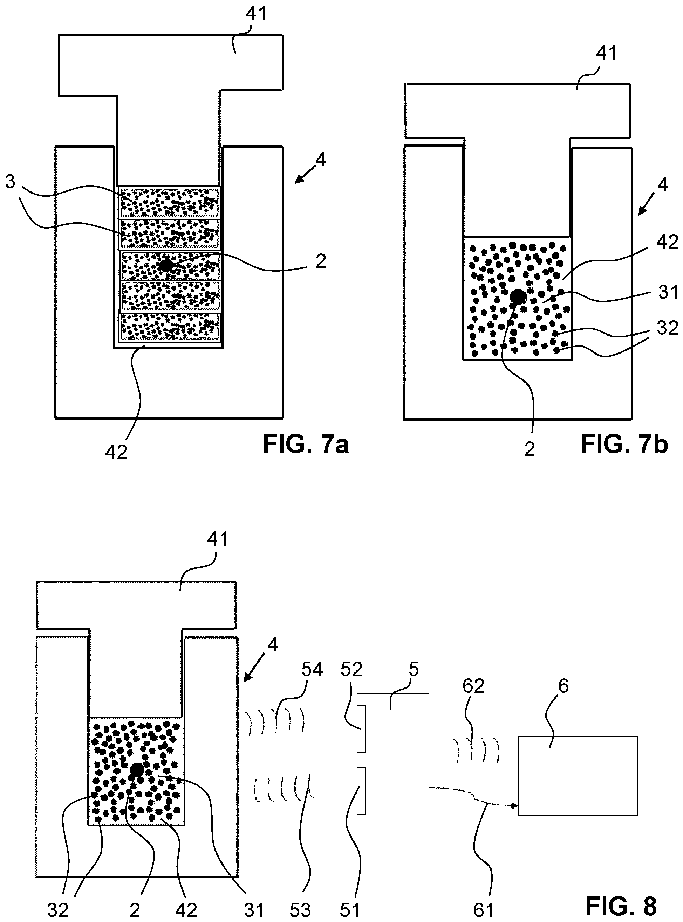

Die Figs. 7a und 7b zeigen nun schematisch ein bevorzugtes Herstellverfahren eines erfindungsgemässen Verbindungsstabes 14 mit integriertem Mikrodraht 2.

Dabei wird ein Pressformwerkzeug 4 verwendet, das z.B. aus Stahl oder einem anderen Material hergestellt ist und zwei Formteile aufweist. Eines der beiden Formteile bildet einen Stempel 41, und gemeinsam bilden die Formteile eine Kavität 42 mit einem hier quadratischen Querschnitt. Gemäss der in Fig. 6 dargestellten zweiten Variante werden die Prepregs 3 zu Vorformlingen bei Raumtemperatur oder bei einer Temperatur weit unterhalb des Schmelzpunktes der Kunststoffmatrix 31 in der Kavität 42 geometrisch exakt angeordnet bzw. gestapelt. Der Prepreg 3 mit dem Mikrodraht 2 wird mittig zu den anderen Prepregs 3 in der Kavität 42 positioniert.

Danach wird der Vorformling mit dem Pressformwerkzeug 4 auf eine Verarbeitungstemperatur oberhalb des Schmelzpunktes der Kunststoffmatrix 31 bzw. des Prepregs 3 erhitzt und durch Schliessen der beiden Formteile auf die Endkontur des Verbindungsstabes 14 verpresst (Fig. 7b) sowie anschliessend abgekühlt. Der integrierte Mikrodraht 2 kann bereits während des Verpressens die Herstelldaten bezüglich des Druckes und der Temperatur des Verbindungsstabes 4 messen und zwecks Aufzeichnung drahtlos übermitteln.

Die Fig. 8 zeigt eine schematische Darstellung der Datenerfassung und -übermittlung eines bevorzugten erfindungsgemässen Implantats während dessen Herstellung. Dies wird beispielhaft anhand des in der Fig. 7b gezeigten Herstellungsschritts erklärt. Das Überwachungssystem umfasst nebst dem passiven Sensor, welcher hier als Mikrodraht 2 vorliegt, ein Auslesegerät 5 mit Sender 51 und Empfänger 52 sowie eine Rechnereinheit 6. Das Auslesegerät 5 ist vom Pressformwerkzeug 4 beabstandet und weist keine kabelgebundene Verbindung dazu auf.

Bereits während der Verpressung durch das Pressformwerkzeug 4 generiert nun der Sender 51 drahtlos eine elektromagnetische Anregung 53, welche auch den im Verbindungsstab 14 befindlichen Mikrodraht 2 erfasst. Aufgrund dieser Anregung 53 wird vom Mikrodraht 2 ein Signal 54 zurückgesendet, welches vom Auslesegerät 5 via den Empfänger 52 zum Beispiel oszillographisch registriert werden kann. Aufgrund der Magnetoelastizität des Mikrodrahts 2 ist das Signal 54 dabei abhängig vom Druck und der Temperatur, welchen der Verbindungsstab 14 und somit der Mikrodraht 2 während der Verpressung ausgesetzt ist. Dementsprechend können basierend auf dem empfangenen Signal 54 vom Auslesegerät 5 Daten bzgl. Druck und Temperatur des Verbindungsstabes 14 ermittelt werden.

Diese ermittelten Daten werden dann mittels einer kabelgebundenen Übertragung 61 oder einer drahtlosen Übertragung 62 an die Rechnereinheit 6 zur anschliessenden Auswertung und Aufzeichnung gesendet.

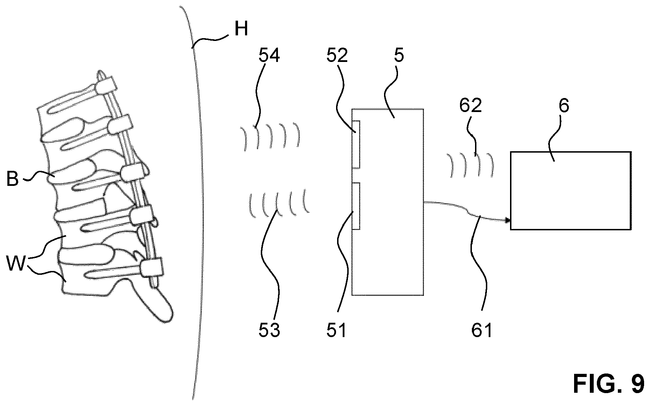

In der Fig. 9 ist eine schematische Darstellung der Datenerfassung und -übermittlung eines erfindungsgemässen Implantats im implantierten Zustand dargestellt. Die Datenerfassung und -übermittlung dient hier zur Überwachung des Heilungsverlaufes. Das Überwachungssystem wird hier in Kombination mit dem in Fig. 1 gezeigten Pedikelfixationssystem 1 gezeigt, bei welchem ein passiver magnetoelastischer Sensor in Form eines Mikrodrahtes 2 im Verbindungsstab 14 integriert ist. Nebst dem Mikrodraht 2 umfasst Überwachungssystem auch hier das ein Auslesegerät 5 mit einem Sender 51 und einem Empfänger 52 sowie eine Rechnereinheit 6. Das Auslesegerät 5 und die Rechnereinheit 6 befinden sich ausserhalb des Körpers und sind durch die Haut H vom Pedikelfixationssystem 1 getrennt.

Der Sender 51 generiert drahtlos eine elektromagnetische Anregung 53, welche u.a. den im Pedikelfixationssystem 1 befindlichen Mikrodraht 2 erfasst. Aufgrund der Anregung 53 wird vom Mikrodraht 2 ein Signal 54 durch die Haut H hindurch zurückgesendet, welches vom Auslesegerät 5 via den Empfänger 52 erfasst wird. Das Signal 54 ist aufgrund der Magnetoelastizität des Mikrodrahts 2 abhängig von den mechanischen Belastungen und der Temperatur, welchen der Verbindungsstab 14 und somit der Mikrodraht 2 ausgesetzt ist. Dementsprechend können vom Auslesegerät 5 basierend auf dem empfangenen Signal 54 Daten bzgl. der mechanischen Belastung und der Temperatur des Verbindungsstabes 14 ermittelt werden.

Diese so ermittelten Daten werden dann mittels einer kabelgebundenen Übertragung 61 oder einer drahtlosen Übertragung 62 an die Rechnereinheit 6 zur anschliessenden Auswertung gesendet. Basierend auf den gemessenen Daten kann der Arzt oder das medizinische Personal die Behandlung zum Bespiel anpassen oder beenden.

**BEZUGSZEICHENLISTE**

| | | | |
|---|---|---|---|
| 1 | Pedikelfixationssystem | | |
| 11 | Pedikelschraube | 5 | Auslesegerät |
| 12 | Schraubenschaft | 51 | Sender |
| 13 | Tulpenkopf | 52 | Empfänger |
| 14 | Verbindungsstab | 53 | Anregung |
| | | 54 | Signal |
| 2 | Mikrodraht | | |
| | | 6 | Rechnereinheit |
| 3 | Prepreg | 61 | Kabelgebundene |
| 31 | Kunststoffmatrix | | Übertragung |
| 32 | Kohlefasern | 62 | Drahtlose Übertragung |
| 4 | Pressformwerkzeug | W | Wirbel |
| 41 | Stempel | B | Bandscheibe |
| 42 | Kavität | H | Haut |

## Patentansprüche

1. Medizinisches Knochenimplantat (1; 11, 14) zur Stabilisierung eines Knochenbereiches, aufweisend
einen aus einem thermoplastischen Kompositmaterial hergestellten Implantatkörper zur Verankerung in oder an einem menschlichen oder tierischen Knochen (W) zwecks Stabilisierung des Knochenbereiches; sowie
zumindest einen Sensor (2) zur Messung von einem oder mehreren Parametern des Heilungsverlaufes und/oder eines Zustands des Implantats (1; 11, 14),
**dadurch gekennzeichnet, dass**
es sich bei dem zumindest einen Sensor um einen passiven magnetoelastischen Sensor (2) handelt.

2. Knochenimplantat (1; 11, 14) nach Anspruch 1, wobei der zumindest eine Sensor (2) zur Messung des einen oder der mehreren Parameter den Barkhausen-Effekt ausnutzt.

3. Knochenimplantat (1; 11, 14) nach Anspruch 1 oder 2, wobei es sich bei dem zumindest einen Sensor um einen Mikrodraht (2) mit einem Durchmesser von bevorzugt 10 µm bis 250 µm handelt.

4. Knochenimplantat (1; 11, 14) nach einem der vorhergehenden Ansprüche, wobei der zumindest eine Sensor (2) zumindest teilweise oder vollständig im Inneren des Implantatkörpers angeordnet ist.

5. Knochenimplantat (1; 11, 14) nach einem der vorhergehenden Ansprüche, wobei das Implantat (1; 11, 14) eine Hauptlängsmittellinie aufweist, und wobei sich der zumindest eine Sensor (2) im Wesentlichen entlang der gesamten Längserstreckung der Hauptlängsmittellinie erstreckt.

6. Knochenimplantat (1; 11, 14) nach Anspruch 5, wobei das Implantat (1; 11, 14) mehrere derartige Sensoren (2) aufweist, welche in regelmässigen Abständen entlang der Hauptlängsmittellinie angeordnet sind.

7. Knochenimplantat (1; 11, 14) nach einem der vorhergehenden Ansprüche, wobei das Implantat (11, 13) mehrere derartige Sensoren (2) aufweist, welche sich parallel zueinander erstrecken.

8. Knochenimplantat (1; 11, 14) nach einem der vorhergehenden Ansprüche, wobei es sich beim Implantat um ein Wirbelsäulenimplantat, wie insbesondere einen Wirbelkörperersatz, einen Bandscheibenersatz, einen Dübel, einen Schrauben-Anker, eine Fixierungsplatte, eine Pedikelschraube (11) oder einen Verbindungsstab (14) eines Pedikelsystems handelt.

9. Knochenimplantat (1; 11, 14) nach einem der Ansprüche 1 bis 7, wobei es sich beim Implantat um ein Traumaimplantat, wie insbesondere einen Schrauben-Anker, einen Dübel, eine Knochenplatte oder eine Knochenschraube, handelt.

10. Knochenimplantat (1; 11, 14) nach einem der vorhergehenden Ansprüche, wobei der eine oder die mehreren Parameter mechanische Belastungen, wie insbesondere Druckkräfte und/oder Biegekräfte, und/der eine Temperatur betreffen.

11. Knochenimplantat (1; 11, 14) nach einem der vorhergehenden Ansprüche, wobei der zumindest eine Sensor (2) eine Temperaturwiderstandsfähigkeit bis mindestens 450 °C hat.

12. Verfahren zur Überwachung des Zustands eines Implantats (1; 11, 14), wobei es sich beim Implantat insbesondere um ein Knochenimplantat (1; 11, 14) nach einem der vorhergehenden Ansprüche handelt, und wobei das Implantat (1; 11, 14) zumindest einen passiven magnetoelastischen Sensor (2) zur Messung von einem oder mehreren Parametern des Zustands des Implantats (1; 11, 14) aufweist,
**dadurch gekennzeichnet, dass**
der Sensor (2) zur Überwachung des Zustands des Implantats (1; 11, 14) während dessen Herstellung und/oder während dessen Lagerung verwendet wird.

13. Verfahren nach Anspruch 12, wobei der Zustand des Implantats (1; 11, 14) ausserdem unmittelbar nach dessen Implantierung in einem menschlichen oder tierischen Körper mittels des zumindest einen Sensors (2) geprüft wird.

14. Verfahren nach Anspruch 12 oder 13, wobei ausserdem der Heilungsverlauf nach der Implantierung des Implantats (1; 11, 14) in einem menschlichen oder tierischen Körper mittels des zumindest einen Sensors (2) überwacht wird.

15. Verfahren nach einem der Ansprüche 12 bis 14, wobei zur Herstellung des Implantats (1; 11, 14) eine Vielzahl von Prepregs (3), insbesondere von unidirektionalen Prepregs, unter Druck und Hitze miteinander verpresst werden, und wobei der zumindest eine Sensor (2) beim Verpressen zwischen den Prepregs (3) angeordnet oder in einem der Prepregs (3) eingebettet ist.
